# FASCICULE DE BREVET EUROPEEN

(11) **EP 1 341 491 B1**
(45) Date de publication et mention de la délivrance du brevet: **08.04.2009**
(21) Numéro de dépôt: 01999334.4
(22) Date de dépôt: 05.12.2001
(51) Int. Cl.: A61F 2/44, A61F 2/30

(54) **IMPLANT INTERSOMATIQUE RACHIDIEN DISTRACTABLE IN SITU COMPORTANT DES POINTS DE PASSAGE DUR**
IN SITU VERLÄNGERBARES ZWISCHENWIRBELIMPLANTAT MIT FESTEN DURCHTRITTSPUNKTEN
SPINAL INTERVERTEBRAL IMPLANT ADJUSTABLE IN SITU COMPRISING HARD PASS POINTS

(30) Priorité: 05.12.2000 FR 0015736
(43) Date de publication de la demande: 10.09.2003
(73) Titulaire: Stryker Spine, 33610 Cestas (FR)
(72) Inventeur: PAPONNEAU, François, F-33170 Gradignan (FR); CONCHY, Frédéric, F-33650 Saint Médard d'Eyrans (FR)
(74) Mandataire: Ahner, Francis
(86) Numéro de dépôt international: PCT/FR2001/003838
(87) Numéro de publication internationale: WO 2002/045625

(56) Documents cités:
- EP-A- 0 567 424
- WO-A-99/56675
- DE-A- 19 622 827
- US-A- 5 702 455
- US-A- 5 916 267

## Description

L'invention concerne les implants de type cage intersomatique ou de remplacement de corps vertébraux destinés à la colonne vertébrale.

Le document WO 99/56675 enseigne un implant destiné au remplacement de corps vertébraux comportant un premier élément central présentant deux filetages dont les pas sont opposés l'un à l'autre. Il comporte en outre deux éléments d'extrémité supérieure et inférieure pouvant se visser sur ledit élément central. De ce fait, l'implant est distractable in situ en mettant en rotation l'élément central. Ainsi, les deux éléments d'extrémité supérieur et inférieur s'éloignent ou se rapprochent l'un de l'autre suivant le sens de rotation de l'élément central. Cependant, en cas de manoeuvre, il est difficile de savoir où en est la distraction. Ceci implique une difficulté de mise en oeuvre lors d'opérations chirurgicales, ce qui a pour conséquence d'allonger le temps opératoire.

Un but de l'invention est de fournir un implant de type cage intersomatique ou de remplacement de corps vertébraux pouvant se régler in situ de manière suffisamment rapide et précise.

Pour cela, on prévoit, selon l'invention, un implant intersomatique rachidien comportant au moins un élément supérieur, un élément inférieur, et un organe intermédiaire apte à coopérer avec les éléments supérieur et inférieur par des moyens de liaison hélicoïdale qui comprennent des moyens formant des points de passage dur lors de la mise en oeuvre de la liaison hélicoïdale.

Ainsi, les moyens formant des points de passage dur permettent une indexation du réglage de la distraction in situ de manière simple et précise pour le chirurgien.

Avantageusement, les moyens de vissage comprennent au moins une came hélicoïdale et un suiveur de came apte à venir en contact avec une surface d'appui de la came.

Avantageusement, la surface d'appui présente des zones formant les points de passage dur.

Avantageusement, les zones formant les points de passage dur comportent des points de passage s'étendant en saillie de la surface d'appui.

Avantageusement, les points hauts de passage sont des bosses.

Avantageusement, l'organe intermédiaire comporte la came.

Avantageusement, l'organe intermédiaire comporte le suiveur de came.

Avantageusement, l'organe intermédiaire est apte à être reçu dans l'un des éléments supérieur et inférieur.

Avantageusement, l'organe intermédiaire est apte à être reçu dans l'autre des éléments supérieur et inférieur.

Avantageusement, l'un des éléments supérieur et inférieur est apte à être reçu dans l'organe intermédiaire.

Avantageusement, l'autre des éléments supérieur et inférieur est apte à être reçu dans l'organe intermédiaire.

Avantageusement, la liaison hélicoïdale entre l'organe intermédiaire et l'élément supérieur a un sens de vissage contraire à celui de la liaison hélicoïdale entre l'élément central et l'élément inférieur.

Avantageusement, l'implant comporte en outre des moyens de blocage en position d'au moins l'un des éléments supérieur et inférieur par rapport à l'organe intermédiaire.

Avantageusement, les moyens de blocage en position comprennent au moins un plot.

Avantageusement, le plot comporte une partie excentrée par rapport à l'axe principal de mise en oeuvre du plot.

Avantageusement, le plot comporte une partie apte à être vissée.

Avantageusement, l'implant comporte des moyens d'ancrage de l'implant dans des plateaux vertébraux.

Avantageusement, les éléments supérieur et inférieur sont aptes à s'imbriquer l'un dans l'autre par complémentarité de forme.

Avantageusement, les éléments supérieur et inférieur s'imbriquent à coulissement.

Avantageusement, chaque élément supérieur et inférieur a une forme générale en « U », les éléments étant aptes à s'imbriquer l'un dans l'autre avec les « U » en opposition.

Avantageusement, l'un au moins des éléments supérieur et inférieur comportant la came, ladite came présente une ouverture en regard de sa surface d'appui.

On prévoit aussi selon l'invention une méthode chirurgicale présentant des étapes de mise en place de l'implant sur le site d'implantation et de réglage in situ dudit implant en mettant en oeuvre les moyens de vissage entre l'élément central et les éléments d'extrémité, les moyens de vissage présentant des moyens formant des points durs de passage.

Avantageusement, la méthode chirurgicale présente en outre une étape de remplissage de l'implant avec une substance favorisant la repousse osseuse.

D'autres caractéristiques et avantages de l'invention apparaîtront lors de la description ci-après d'un mode préféré de réalisation de l'invention ainsi que d'une variante donnée à titre d'exemples non limitatifs. Aux dessins annexée :
- la figure 1 est une vue en trois dimensions d'un premier mode de réalisation de l'invention en position de hauteur minimale ;
- la figure 2 est une vue en trois dimensions du premier mode de réalisation de l'invention en position de hauteur maximale ;
- la figure 3 est une vue en trois dimensions de l'une des bases du premier mode de réalisation de la figure 1 ;
- la figure 4 est une vue en trois dimensions de l'élément intermédiaire du premier mode de réalisation de la figure 1 ;
- la figure 5 est une vue en trois dimensions d'un second mode de réalisation de l'invention en position de hauteur minimale ;
- la figure 6 est une vue en trois dimensions du second mode de réalisation de l'invention en position de hauteur maximale ;
- la figure 7 est une vue en trois dimensions de l'élément intermédiaire du second mode de réalisation de la figure 5 ;
- la figure 8 est une vue en trois dimensions de l'une des bases du second mode de réalisation de la figure 5 ; et
- la figure 9 est une vue en trois dimensions montrant l'agencement des deux éléments de base du second mode de réalisation de la figure 5.

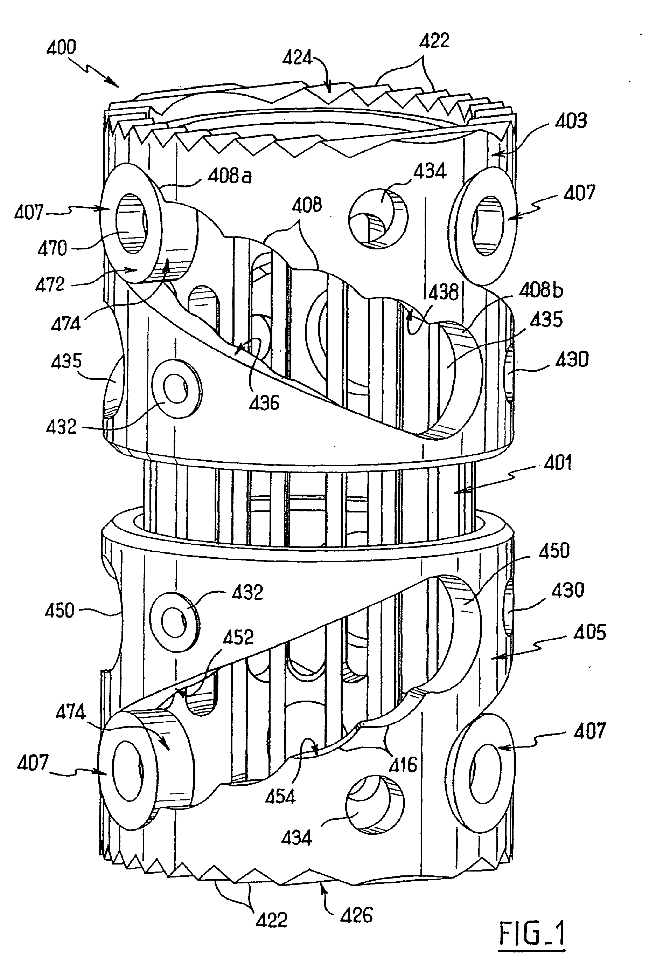

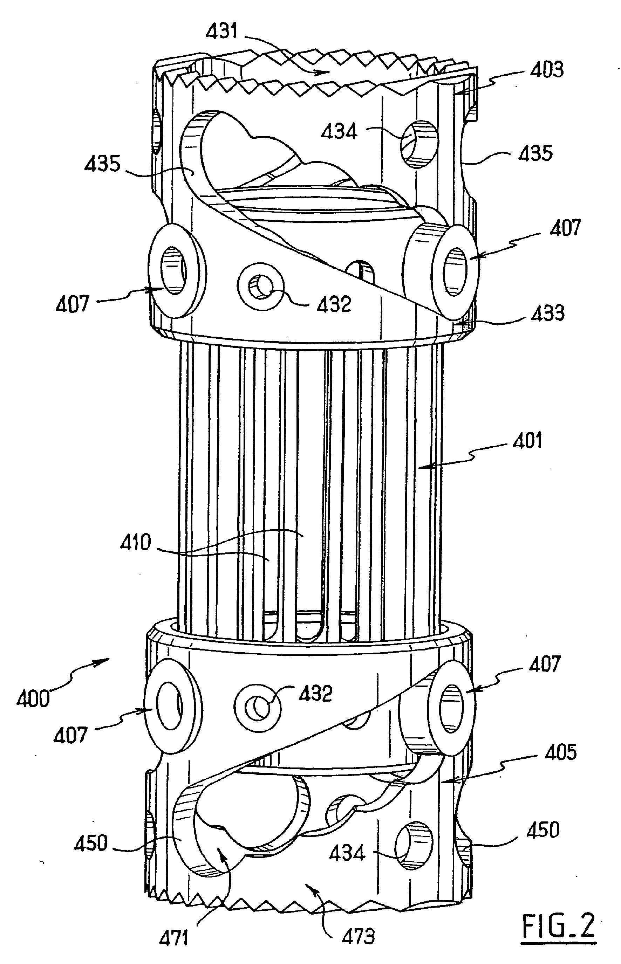

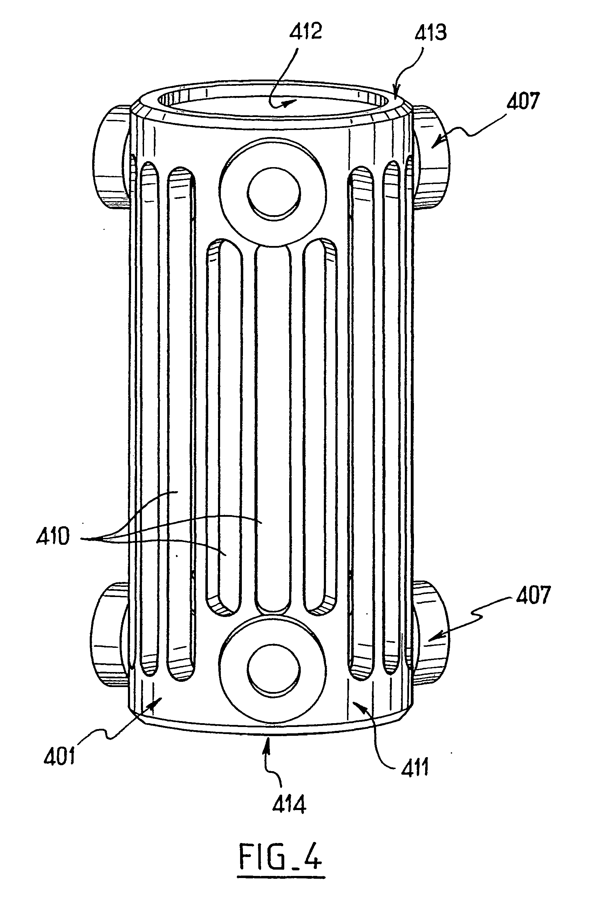

On va décrire un premier mode de réalisation de l'invention en référence aux figures 1 à 4. L'implant 400 de type cage intersomatique de ce présent mode de réalisation comporte des éléments principaux suivantes : une base supérieure 403, une base inférieure 407 ainsi qu'un élément intermédiaire 401 apte à être reçu à coulissement et à rotation à l'intérieur des bases supérieure et inférieure.

L'élément intermédiaire 401 est un tube présentant un diamètre interne et un diamètre externe. La paroi du tube comporte une pluralité d'ouvertures longitudinales 410 similaires à des lumières. Ces ouvertures 410 ont leur grande dimension parallèle à une génératrice du tube formant l'élément intermédiaire et sont parallèles les unes par rapport aux autres. Les ouvertures 410 s'étendent radialement de la face externe 411 à la face interne 412, faces délimitant l'épaisseur du tube formant l'élément intermédiaire 401. Cet élément intermédiaire 401 présente donc un creux intérieur qui s'étend d'une face supérieure 413 à une face inférieure 414 et qui est délimitée radialement par la face interne 412. Le creux intérieur est apte à recevoir toute substance ostéoconductrice ou ostéoinductrice qui favorise la fusion osseuse.

D'autre part, l'élément intermédiaire 401 comporte une pluralité de plots 407 s'étendant en saillie de la face externe 411 de l'élément intermédiaire 401 selon une direction radiale, vers l'extérieur. Les plots 407 sont uniformément répartis sur la circonférence du tube en deux groupes situés respectivement prés des faces d'extrémité supérieure 413 et inférieure 414. Chaque groupe comporte, ici, trois plots 407. Chacun des plots 407 comprend une surface 474 dite de contact, une face frontale plane 472 perpendiculaire à la surface 474, ainsi que des moyens de mise en oeuvre 470. La surface 474 est une surface de révolution, ici cylindrique, alors que la face 472 est plane et circulaire. La hauteur des plots 407 est telle que, une fois l'implant 400 monté, la face 472 affleure la face externe 473 des bases 403 et 405, bases que l'on va décrire maintenant.

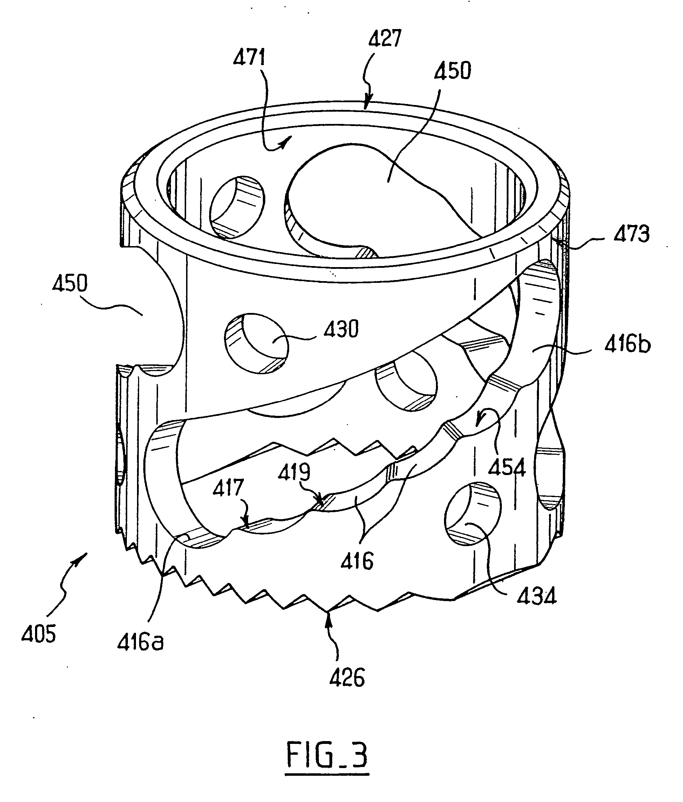

En référence à la figure 3, la base inférieure 405 est, ici, un tube présentant une face supérieure 427, une face inférieure 426, une face externe 473 ainsi qu'une face interne 471. Le diamètre interne du tube est sensiblement équivalent au diamètre externe de celui formant l'élément intermédiaire 401. Ainsi, lors du montage de l'implant 400, la face 471 est en contact avec la face 411 et l'élément intermédiaire 401 est monté mobile à coulissement et à rotation avec la base 405. La face supérieure 427 est perpendiculaire à l'axe de révolution du tube formant la base 405. La face inférieure 426, ici sensiblement parallèle à la face 427, comporte une pluralité de dents 422, ici profilées avec une section triangulaire et parallèles les unes aux autres. Les dents 422 forment dans le cas présent des moyens d'ancrage dans les plateaux vertébraux avec lesquels la face 426 est apte à venir en contact.

La face externe 473 comporte une pluralité d'ouvertures 450 de forme hélicoïdale, traversant l'épaisseur de la paroi du tube formant la base 405 et uniformément répartie sur la circonférence de la base 405. Ici, elles sont au nombre de trois. Chaque ouverture 450 comporte une face lisse 452 située du côté de la face plane 427 de la base 405 et formant une rampe unique. L'ouverture 450 comporte, en outre, une surface 454 opposée, en regard et sensiblement parallèle à la face 452. Cette surface 454 dite de contact comporte un groupe de plusieurs rampes 416, ici au nombre de six, de forme concave, dont le rayon de courbure est sensiblement égal au rayon de la surface 474 des plots 407 avec laquelle les rampes 416 sont aptes à venir en contact lors de la mise en oeuvre de l'implant 400. Chaque rampe 416 comporte une extrémité basse 417 et une extrémité haute 419. L'agencement des rampes 416 au sein du groupe est tel qu'une extrémité haute d'une rampe forme l'extrémité basse de la rampe suivante, les rampes 416a et 416b dites d'extrémité du groupe n'ont pas d'extrémité basse et haute respectivement car leur surface se poursuit avec le même rayon de courbure de manière à venir rejoindre tangentiellement la face 452 et compléter ainsi l'ouverture 450.

D'autre part, la face externe 473 de la base 405 comporte une pluralité d'orifices 430 de section circulaire, filetés (le filetage n'est pas représenté), situés près de la face 427, uniformément répartis sur la circonférence de la base 405 et aptes à recevoir un verrou 432 par vissage. Ce dernier est plus long que l'épaisseur de la paroi de la base 405 de manière à pouvoir s'enfiler dans l'une des ouvertures 410 de l'élément intermédiaire 401. Le verrou 432 a pour rôle de sécuriser le montage de l'implant 400, comme on le verra plus loin dans la description.

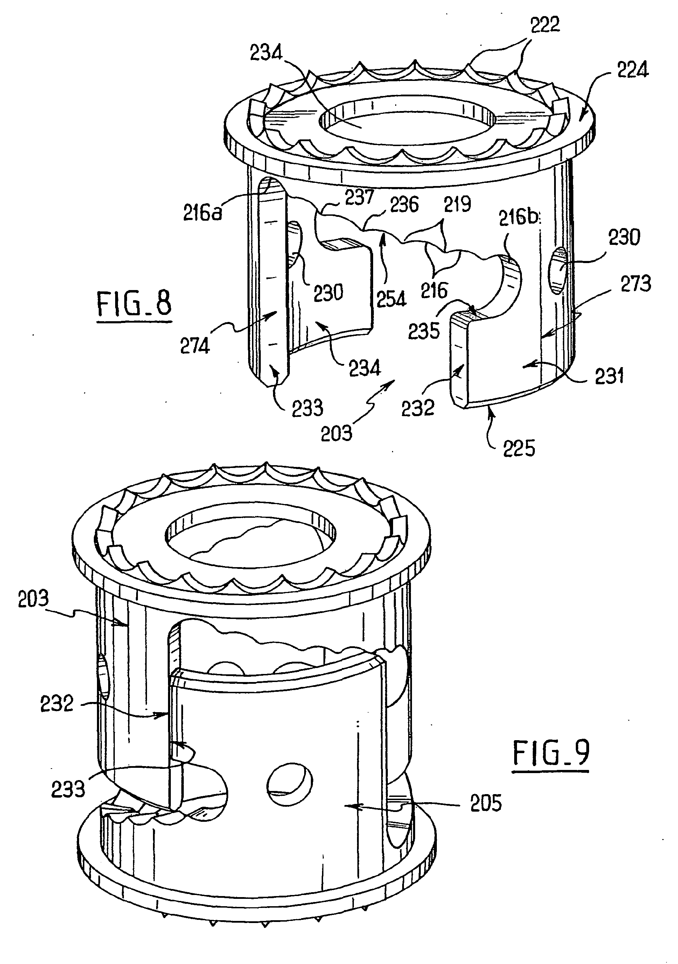

De même, la face externe 473 de la base 405 comporte une pluralité d'orifices 434 de section circulaire, lisses, situés près de la face 426 de la base 405 et uniformément répartis sur la circonférence de la base 405. Ces orifices permettent d'améliorer la communication entre l'extérieur et l'intérieur de l'implant 400 lorsque ce dernier est en configuration haute, comme cela est illustré à la figure 8.

La base supérieure 403 est la symétrique en miroir, selon un plan transversal perpendiculaire à l'axe de révolution de la base, de la base inférieure 405 que l'on vient de décrire en détail. La base 403 comporte une pluralité d'ouvertures 435 symétriques des ouvertures 450 : chaque ouverture 435 comporte une face 436 uniforme formant une rampe unique, une surface 438 dite de contact opposé en regard de la face 436 et comprenant un groupe d'une pluralité de rampes 408 similaires aux rampes 416. Le groupe comprend des rampes 408a et 408b dites d'extrémité dont la surface se prolonge selon le même rayon de courbure pour venir rejoindre tangentiellement la face 436. La base 403 comporte, en outre, une face 424 comportant des moyens d'ancrage 422, ici, des dents similaires à celles de la face 426 précédemment décrites.

Lors de l'intervention chirurgicale, le chirurgien établit la voie d'abord puis prépare le site d'implantation. Il monte alors l'implant 400 en choisissant l'élément intermédiaire 401 ainsi qu'une base 403 et une base 405. Il assemble les bases sur l'élément intermédiaire. Il met en place les différents plots 407 sur l'élément 401 à travers les ouvertures hélicoïdales 435 et 450. Il remplit l'intérieur d'un implant 400 ainsi constitué avec une substance ostéoinductrice ou ostéoconductrice. Il met ensuite en place l'implant 400 dans le site d'implantation. Il règle la hauteur de l'implant 400 en faisant tourner l'élément intermédiaire 401 alors que les bases 403 et 405 sont immobiles du fait des moyens d'ancrage impactés dans les plateaux vertébraux délimitant le site d'implantation. La mise en rotation de l'implant 401, du fait de la liaison hélicoïdale reliant l'élément intermédiaire 401 à chaque base 403,405, fait se déplacer les plots 407 dans leurs ouvertures respectives 435 et 450, leur surface 474 passant d'une rampe 416, 408 à l'autre, ce qui permet le réglage de la hauteur de l'implant. Une fois la hauteur de distraction désirée obtenue, le chirurgien sécurise l'implant 400 en position de distraction en mettant en oeuvre les moyens de blocage en position 432, puis referme ensuite sa voie d'abord. Grâce aux discontinuités entre les rampes 416, le chirurgien sait que chaque passage de rampe correspond à une augmentation ou une diminution de hauteur donnée fixe. Cette indexation du réglage de la hauteur permet au chirurgien de savoir où en est la distraction du site d'implantation.

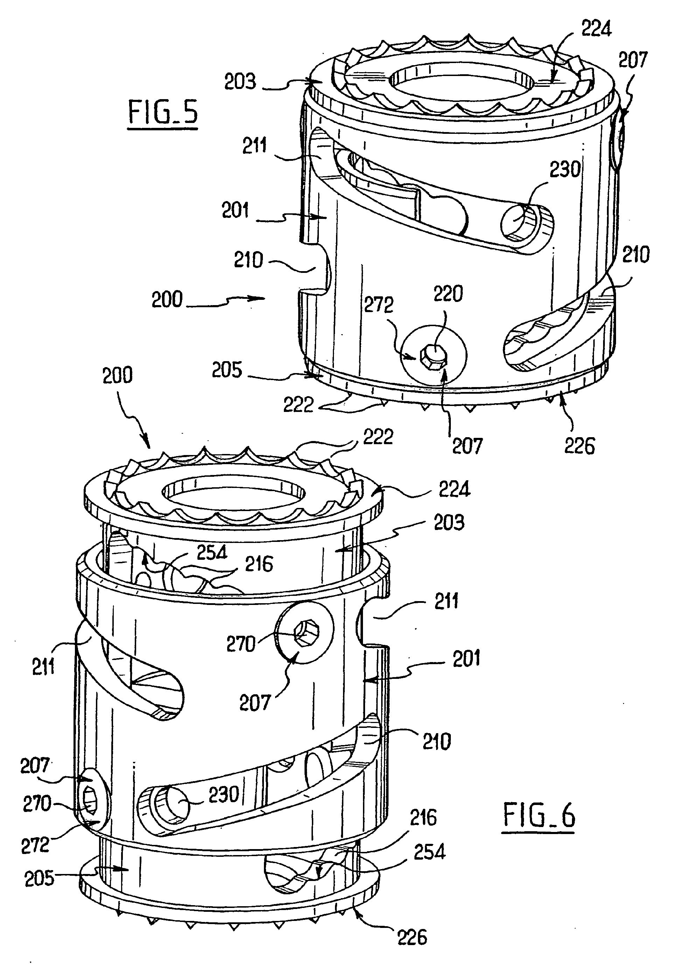

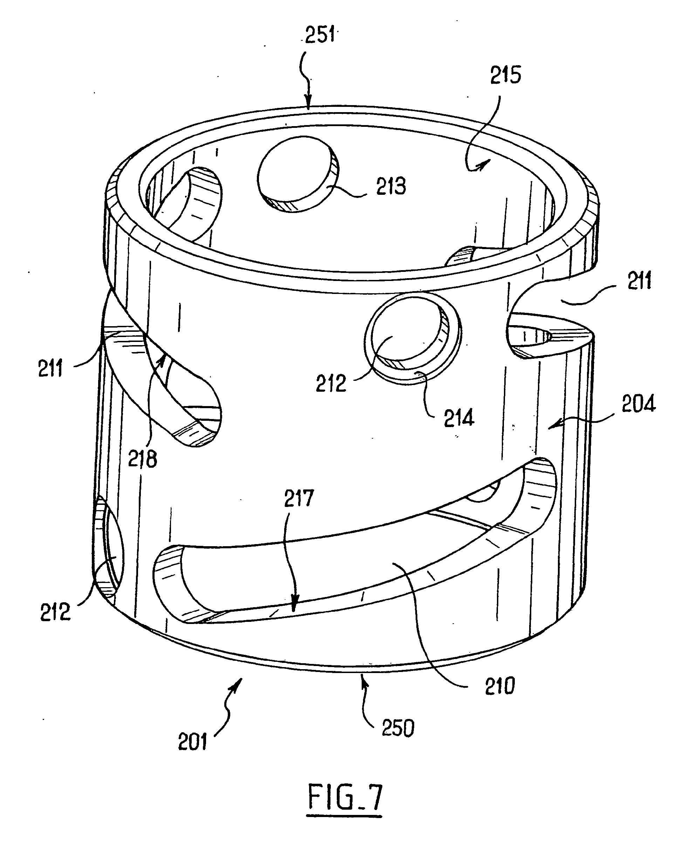

En référence aux figures 5 à 9, on va décrire un second mode de réalisation de l'invention. L'implant 200 de type cage intersomatique de ce présent mode de réalisation comporte trois éléments principaux : une base supérieure 203, une base inférieure 205 ainsi qu'un élément intermédiaire 201. Les bases supérieure 203 et inférieure 205 sont aptes à être reçues à coulissement et à rotation à l'intérieur de l'élément intermédiaire 201, pour former une liaison hélicoïdale comme dans le premier mode de réalisation précité.

L'élément intermédiaire 201 est un tube présentant un diamètre interne et un diamètre externe. La paroi du tube comporte une première pluralité d'ouvertures 210, de forme hélicoïdale dont l'hélice se visse dans le sens de rotation des aiguilles d'une montre vers le bas lorsque son axe est vertical. Ces ouvertures 210, ici au nombre de deux, sont uniformément réparties sur la circonférence du tube et situées près de la face d'extrémité inférieure 250. De même, la paroi du tube comporte une deuxième série d'ouvertures hélicoïdales 211 dont l'hélice se visse dans le sens inverse des aiguilles d'une montre vers le bas lorsque son axe est vertical. Ces ouvertures 211, ici aussi au nombre de deux, sont uniformément réparties le long de la circonférence du tube et situées près de la face d'extrémité supérieure 251. Les ouvertures 210 et 211 présentent chacune une surface d'appui 217, 218 respectivement. Les surfaces d'appui sont aptes à venir en contact avec des moyens de blocage en position (non représentés sur les figures), comme on le verra plus loin dans la description.

D'autre part, près des faces d'extrémité supérieure 251 et inférieure 250, l'élément intermédiaire 201 comporte une pluralité d'orifices circulaires 212 uniformément réparties sur la circonférence du tube, ici au nombre de deux. Ces orifices 212 sont traversants de la face externe 204 à la face interne 215. Chacun des orifices 212 comporte une partie filetée 213 ainsi qu'un lamage 214 dont le diamètre est supérieur à celui de la partie filetée 213. Le filet 213 est apte à venir coopérer avec celui présent sur un plot 207 qui sera décrit ultérieurement. De même, le lamage 214 est apte à recevoir la tête du plot 207.

Chacun des plots 207 comprend une face sensiblement plane et circulaire 272 dans laquelle sont aménagés des moyens de mise en oeuvre 270, ici sous la forme d'une empreinte hexagonale. Une fois mis en place, le plot 207 affleure la face externe 204 de l'élément intermédiaire 201. D'autre part, la longueur de ce plot 207 est très supérieure à l'épaisseur du tube formant l'élément intermédiaire. Ceci pour pouvoir venir en contact avec une partie 254 des bases supérieure 203 et inférieure 205, que l'on va décrire maintenant.

En référence à la figure 8, on ne décrira que la base supérieure 203, la base inférieure 205 étant la symétrique en miroir selon un plan transversal perpendiculaire à l'axe de révolution de la base, de la base supérieure 203 que l'on va maintenant décrire en détail. La base supérieure 203 est, ici, un tube présentant une face supérieure 224, une face inférieure 225, une face externe 231 ainsi qu'une face interne 234. Le diamètre externe du tube est sensiblement équivalent au diamètre interne de celui formant l'élément intermédiaire 201. Ainsi, lors du montage de l'implant 200, la face 231 est en contact avec la face 215 et la base supérieure 203 est ainsi montée mobile à coulissement et à rotation avec l'élément intermédiaire 201. La face inférieure 225 est perpendiculaire à l'axe de révolution du tube formant la base 203. La face supérieure 224, ici sensiblement parallèle à la face 225, comporte pluralité de dents 222, ici profilées avec une section triangulaire présentant des cotés concaves et uniformément réparties sur un cercle ayant pour centre, l'axe de révolution du tube formant la base 203 et de diamètre correspondant à la moyenne des diamètres interne et externe dudit tube formant ladite base 203. Les dents 222 forment dans le cas présent des moyens d'ancrage dans les plateaux vertébraux avec lesquels la face 224 est à venir en contact. D'autre part, la face 224 présente un orifice 234 circulaire de centre l'axe de révolution du tube formant la base 223.

Le corps de la base comporte deux prolongements formant des branches 273 et 274. Ces deux prolongements sont symétriques l'un de l'autre, suivant l'axe de révolution du tube formant la base 203. Les deux prolongements 273 et 274 délimitent de part et d'autre de l'axe deux ouverture qui sont chacune ouvertes vers la face d'extrémité inférieure 225 de la base 203, ouvertures comportant une première face latérale 233 sensiblement verticale, une face supérieure 254, une face 235, ainsi qu'une deuxième face latérale 232 sensiblement verticale et sensiblement parallèle à la première face latérale 233. La face supérieure 254 dite surface de contact ou surface d'appui comporte un groupe de plusieurs rampes 216, ici au nombre de sept, de forme concave dont le rayon de courbure est sensiblement égal au rayon de la partie des plots 207 avec laquelle la face 254 est apte à venir en contact lors de la mise en oeuvre de l'implant 200. Chaque rampe 216 comporte une extrémité basse 236 et une extrémité haute 237. L'agencement des rampes 216 au sein du groupe est telle que l'extrémité haute d'une rampe forme l'extrémité basse de la rampe suivante ; les rampes 216a et 216b dites d'extrémité du groupe n'ont pas d'extrémités haute et basse respectivement, car leur surface se poursuit avec le même rayon de courbure pour rejoindre tangentiellement la face 233 et la face 235 respectivement. Les extrémités haute et basse 237, 236 forment une pluralité de bosses 219.

D'autre part, la branche 273, tout comme la branche 274, comporte un orifice traversant radialement 230 de section circulaire. Cet orifice 230 est apte à recevoir les moyens de blocage en position (non représentés sur les figures) de l'implant 200.

Un tel agencement des branches 273 et 274 de la base 203 ainsi que ses homologues de la base 205 qui est symétrique en miroir de la base 203 comme indiqué précédemment, permet l'imbrication des deux bases lors du montage de l'implant et de son utilisation, comme illustré à la figure 9. Les deux bases 203 et 205 sont montées à coulissement l'une par rapport à l'autre, les différentes faces 232 d'une des bases étant aptes à venir en contact avec les différentes faces 233 de l'autre base respectivement. Au cours de cette imbrication, chaque prolongement pénètre dans l'une des ouvertures.

Lors de l'intervention chirurgicale, le chirurgien comme précédemment, établit la voie d'abord puis prépare le site d'implantation. Il monte alors l'implant 200 en choisissant un élément intermédiaire 201 ainsi qu'une base 203 et une base 205. Les différents plots 207 étant déjà placés sur l'élément intermédiaire 201, au sein des orifices 212, il met en place les deux bases en les insérant au sein de l'élément intermédiaire 201, tel qu'illustré à la figure 5, les plots 207 étant en contact avec les rampes 216a de la base supérieure 203 et de son homologue symétrique de la base 205. L'implant est en configuration basse alors. Il met ensuite en place les moyens de blocage en position non représentés à travers les lumières 211 et 210 respectivement, dans les orifices 230 des bases 203 et 205. Les moyens de blocage en position peuvent être des plots présentant un filetage venant coopérer avec un filetage aménagé dans l'orifice 230, ou bien des plots aptes à être montés à rotation dans l'orifice 230 dont la tête reçue dans les lumières 210, 211, est excentrée par rapport à l'axe de l'orifice 230. Une fois l'implant 200 monté, le chirurgien remplit l'intérieur de l'implant avec une substance ostéoinductrice ou ostéoconductrice. Il met ensuite en place l'implant 200 dans le site d'implantation. Puis, il règle la hauteur de l'implant en faisant tourner l'élément intermédiaire 201 alors que les bases 203 et 205 sont immobiles par rapport aux vertèbres respectives, du fait des moyens d'ancrage impactés dans les plateaux vertébraux délimitant le site d'implantation. La mise en rotation de l'élément intermédiaire 201 fait se déplacer les plots 207 le long des rampes 216 de chacune des ouvertures des bases 203 et 205. Le chirurgien met en oeuvre ensuite les moyens de blocage en position reçus à travers les lumières 210 et 211 pour bloquer l'implant en position. Le chirurgien referme ensuite la voie d'abord.

Bien entendu, on pourra apporter à la présente invention de nombreuses modifications sans sortir du cadre de celle-ci.

Les faces comportant les moyens d'ancrage pourront être inclinées par rapport à un plan perpendiculaire principal de l'implant.

On pourra imaginer tout système de came avec suiveur de came autre que celui décrit précédemment sans sortir de la présente invention.

On pourra aussi prévoir de réaliser un implant présentant des éléments supérieur et inférieur aptes à s'imbriquer l'un dans l'autre par complémentarité de forme, comme par exemple illustré sur la figure 9, sans avoir de liaisons hélicoïdales entre l'organe intermédiaire et les éléments supérieur et inférieur présentant des moyens formant des points de passage dur.

## Revendications

1. Implant intersomatique rachidien comportant au moins un élément supérieur (203;403), un élément intérieur (205;405), un organe intermédiaire (201:401), et des moyens de liaison, hélicoïdale (207,254;407,435,450) par lesquels l'organe intermédiaire est apte à coopérer avec les éléments supérieur et inférieur, **caractérisé en ce que** les moyens de liaison hélicoïdale comprennent des moyens (216;408,416) formant des points de passage dur lors de la mise en oeuvre de la liaison hélicoïdale.

2. Implant selon la revendication 1, **caractérisé en ce que** les moyens de liaison hélicoïdale comprennent au moins une came (254;435,450) et un suiveur de came (207;407) apte à venir en contact avec une surface d'appui (254;438,454) de la came.

3. Implant selon la revendication 2, **caractérisé en ce que** la surface d'appui présente des zones formant les points de passage dur (236,237;417,419).

4. Implant selon la revendication 2 ou 3, **caractérisé en ce que** les zones formant les points de passage dur comportent des points de passage (236,237;417,419) s'étendant en saillie de la surface d'appui.

5. Implant selon la revendication 4, **caractérisé en ce que** les points hauts de passage sont des bosses (219;417,419).

6. Implant selon l'une des revendications 2 à 5, **caractérisé en ce que** l'organe intermédiaire comporte la came.

7. Implant selon l'une des revendications 2 à 5, **caractérisé en ce que** l'organe intermédiaire comporte le suiveur de came (207;407).

8. Implant selon l'une des revendications précédentes, **caractérisé en ce que** l'organe intermédiaire (401) est apte à être reçu dans l'un des éléments supérieur (403) et inférieur (405).

9. Implant selon la revendication 8, **caractérisé en ce que** l'élément central est apte à être reçu dans l'autre des éléments supérieur et inférieur.

10. Implant selon l'une des revendications 1 à 7, **caractérisé en ce que** l'un des éléments supérieur (203) et inférieur (205) est apte à être reçu dans l'organe intermédiaire (201).

11. Implant selon la revendication 10, **caractérisé en ce que** l'autre des éléments supérieur et inférieur est apte à être reçu dans l'organe intermédiaire.

12. Implant selon l'une des revendications précédentes, **caractérisé en ce que** la liaison hélicoïdale entre l'organe intermédiaire et l'élément supérieur a un sens de vissage contraire à celui de la liaison hélicoïdale entre l'élément central et l'élément inférieur.

13. Implant selon l'une des revendications précédentes, **caractérisé en ce qu'**il comporte en outre des moyens de blocage en position (432) d'au moins l'un des éléments supérieur et inférieur par rapport à l'organe intermédiaire.

14. Implant selon la revendication 13, **caractérisé en ce que** les moyens de blocage en position comprennent un plot (432).

15. Implant selon la revendication 14, **caractérisé en ce que** le plot comporte une partie excentrée par rapport à l'axe principal de mise en oeuvre du plot.

16. Implant selon la revendication 14, **caractérisé en ce que** le plot comporte une partie apte à être vissée.

17. Implant selon l'une des revendications précédentes, **caractérisé en ce qu'**il comporte des moyens d'ancrage (222;422) de l'implant dans des plateaux vertébraux.

18. Implant selon l'une des revendications précédentes, **caractérisé en ce que** les éléments supérieur (203) et inférieur (205) sont aptes à s'imbriquer par complémentarité de forme.

19. Implant selon la revendication 18 **caractérisé en ce que** les éléments supérieur et inférieur s'imbriquent à coulissement.

20. Implant selon la revendication 18 ou 19, **caractérisé en ce que** chaque élément inférieur et supérieur a une forme générale en « U », les éléments étant aptes à s'imbriquer l'un dans l'autre avec les « U » en opposition.

21. Implant selon la revendication 18, 19 ou 20 et selon la revendication 7, **caractérisé en ce que**, l'un au moins des éléments supérieur et inférieur comportant la came (254), ladite came présente une ouverture en regard de sa surface d'appui (254).

## Claims

1. Spinal intervertebral implant comprising at least an upper element (203; 403), a lower element (205; 405), an intermediate member (201; 401), and helical linking means (207, 254; 407, 435, 450) via which the intermediate member can cooperate with the upper and lower elements, **characterized in that** the helical linking means include means (216; 408, 416) forming hard pass points when the helical linkage is carried out.

2. Implant according to Claim 1, **characterized in that** the helical linking means comprise at least a cam (254; 435, 450) and a cam follower (207; 407) which can come into contact with a bearing surface (254; 438, 454) of the cam.

3. Implant according to Claim 2, **characterized in that** the bearing surface has zones forming the hard pass points (236, 237; 417, 419).

4. Implant according to Claim 2 or 3, **characterized in that** the zones forming the hard pass points comprise pass points (236, 237; 417, 419) protruding from the bearing surface.

5. Implant according to Claim 4, **characterized in that** the high pass points are bosses (219; 417, 419).

6. Implant according to one of Claims 2 to 5, **characterized in that** the intermediate member comprises the cam.

7. Implant according to one of Claims 2 to 5, **characterized in that** the intermediate member comprises the cam follower (207; 407).

8. Implant according to one of the preceding claims, **characterized in that** the intermediate member (401) can be received in one of the upper (403) and lower (405) elements.

9. Implant according to Claim 8, **characterized in that** the central element can be received in the other of the upper and lower elements.

10. Implant according to one of Claims 1 to 7, **characterized in that** one of the upper (203) and lower (205) elements can be received in the intermediate member (201).

11. Implant according to Claim 10, **characterized in that** the other of the upper and lower elements can be received in the intermediate member.

12. Implant according to one of the preceding claims, **characterized in that** the helical linkage between the intermediate member and the upper element has a direction of screwing counter to that of the helical linkage between the central element and the lower element.

13. Implant according to one of the preceding claims, **characterized in that** it additionally comprises means (432) for blocking in position at least one of the upper and lower elements relative to the intermediate member.

14. Implant according to Claim 13, **characterized in that** the means for blocking in position comprise a stud (432).

15. Implant according to Claim 14, **characterized in that** the stud comprises a part which is eccentric in relation to the main axis of use of the stud.

16. Implant according to Claim 14, **characterized in that** the stud comprises a part which can be screwed.

17. Implant according to one of the preceding claims, **characterized in that** it comprises means (222; 422) for anchoring the implant in the vertebral plateaus.

18. Implant according to one of the preceding claims, **characterized in that** the upper (203) and lower (205) elements can fit into one another through being of complementary shape.

19. Implant according to Claim 18, **characterized in that** the upper and lower elements fit into one another with sliding.

20. Implant according to Claim 18 or 19, **characterized in that** each lower and upper element has a general U-shape, the elements being able to fit into one another with the U-shapes in opposition.

21. Implant according to Claim 18, 19 or 20 and according to Claim 7, **characterized in that**, with at least one of the upper and lower elements comprising the cam (254), said cam has an opening facing its bearing surface (245).

## Patentansprüche

1. Zwischenwirbelimplantat, umfassend mindestens ein oberes Element (203; 403), ein unteres Element (205; 405), ein Zwischenglied (201; 401) und spiralförmige Verbindungsmittel (207, 254; 407, 435, 450), über die das Zwischenglied in der Lage ist, mit dem oberen und unteren Element zusammenzuwirken, **dadurch gekennzeichnet, dass** die spiralförmigen Verbindungsmittel Mittel (216; 408, 416) umfassen, die bei der Durchführung der spiralförmigen Verbindung feste Durchtrittspunkte bilden.

2. Implantat nach Anspruch 1, **dadurch gekennzeichnet, dass** die spiralförmigen Verbindungsmittel mindestens eine Kurve (254; 435, 450) und einen Kurvenfolger (207; 407) umfassen, die in der Lage sind, mit einer Auflagefläche (254; 438, 454) der Kurve in Berührung zu kommen.

3. Implantat nach Anspruch 2, **dadurch gekennzeichnet, dass** die Auflagefläche Bereiche aufweist, welche die festen Durchgangspunkte (236, 237; 417, 419) bilden.

4. Implantat nach Anspruch 2 oder 3, **dadurch gekennzeichnet, dass** die Bereiche, welche die festen Durchgangspunkte bilden, Durchgangspunkte (236, 237; 417, 419) umfassen, die sich von der Auflagefläche vorspringend erstrecken.

5. Implantat nach Anspruch 4, **dadurch gekennzeichnet, dass** die oberen Durchgangspunkte Erhebungen (219; 417, 419) sind.

6. Implantat nach einem der Ansprüche 2 bis 5, **dadurch gekennzeichnet, dass** das Zwischenglied die Kurve umfasst.

7. Implantat nach einem der Ansprüche 2 bis 5, **dadurch gekennzeichnet, dass** das Zwischenglied den Kurvenfolger (207; 407) umfasst.

8. Implantat nach einem der vorhergehenden Ansprüche, **dadurch gekennzeichnet, dass** das Zwischenglied (401) entweder in dem oberen Element (403) oder in dem unteren Element (405) aufgenommen werden kann.

9. Implantat nach Anspruch 8, **dadurch gekennzeichnet, dass** das mittlere Element in dem anderen oberen bzw. unteren Element aufgenommen werden kann.

10. Implantat nach einem der Ansprüche 1 bis 7, **dadurch gekennzeichnet, dass** das entweder das obere Element (203) oder das untere Element (205) in dem Zwischenglied (201) aufgenommen werden kann.

11. Implantat nach Anspruch 10, **dadurch gekennzeichnet, dass** das andere des oberen bzw. unteren Elements in dem Zwischenglied aufgenommen werden kann.

12. Implantat nach einem der vorhergehenden Ansprüche, **dadurch gekennzeichnet, dass** die spiralförmige Verbindung zwischen dem Zwischenglied und dem oberen Element eine Schraubrichtung aufweist, die zu derjenigen der spiralförmigen Verbindung zwischen dem mittleren Element und dem unteren Element gegenläufig ist.

13. Implantat nach einem der vorhergehenden Ansprüche, **dadurch gekennzeichnet, dass** es außerdem Mittel zum Blockieren der Position (432) mindestens eines des oberen bzw. unteren Elements gegenüber dem Zwischenglied umfasst.

14. Implantat nach Anspruch 13, **dadurch gekennzeichnet, dass** die Positionsblockiermittel ein Kontaktstück (432) umfassen.

15. Implantat nach Anspruch 14, **dadurch gekennzeichnet, dass** das Kontaktstück einen im Verhältnis zur Haupteinsatzachse des Kontaktstücks außermittiges Teil umfasst.

16. Implantat nach Anspruch 14, **dadurch gekennzeichnet, dass** das Kontaktstück einen schraubbaren Teil umfasst.

17. Implantat nach einem der vorhergehenden Ansprüche, **dadurch gekennzeichnet, dass** es Mittel (222; 422) zum Verankern des Implantats in Wirbelkörpern umfasst.

18. Implantat nach einem der vorhergehenden Ansprüche, **dadurch gekennzeichnet, dass** das obere Element (203) und das untere Element (205) sich formschlüssig verschachteln können.

19. Implantat nach Anspruch 18, **dadurch gekennzeichnet, dass** die oberen und unteren Elemente sich gleitend verschachteln.

20. Implantat nach Anspruch 18 oder 19, **dadurch gekennzeichnet, dass** jedes untere und obere Element allgemein "U"-förmig ist, wobei die Elemente sich ineinander verschachteln können, so dass "U"-Teile entgegengesetzt liegen.

21. Implantat nach Anspruch 18, 19 oder 20 und nach Anspruch 7, **dadurch gekennzeichnet, dass** wenn mindestens eines der oberen und unteren Elemente die Kurve (254) umfasst, diese Kurve eine Öffnung gegenüber ihrer Auflagefläche (254) aufweist.
